(19) Europäisches Patentamt
European Patent Office
Office européen des brevets

(11) Veröffentlichungsnummer: **0 136 636**
**A2**

(12) **EUROPÄISCHE PATENTANMELDUNG**

(21) Anmeldenummer: **84111316.0**

(22) Anmeldetag: **22.09.84**

(51) Int. Cl.$^4$: **C 07 D 239/06**
A 01 N 43/54

(30) Priorität: **06.10.83 JP 185854/83**

(43) Veröffentlichungstag der Anmeldung:
**10.04.85 Patentblatt 85/15**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kozo, Shiokawa**
**210-6, Shukugawara Tama-ku**
**Kawasaki-shi Kanagawa(JP)**

(72) Erfinder: **Shinzo, Kagabu**
**432-131-105, Terada-machi**
**Hachioji-shi Tokyo(JP)**

(72) Erfinder: **Shinichi, Tsuboi**
**3-26-1, Hirayama**
**Hino-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Nitromethylen-tetrahydropyrimidin-Derivate, Verfahren zu ihrer Herstellung sowie insektizide, mitizide, tickizide und nematizide Mittel.**

(57) Die vorliegende Erfindung beschreibt
(1) ein Nitromethylen-tetrahydropyrimidin-Derivat der allgemeinen Formel (I)

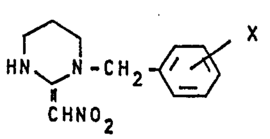

.(I)

in der X für Halogen steht,
(2) Verfahren zur Herstellung des Nitromethylen-tetrahydropyrimidin-Derivats der allgemeinen Formel (I),
(3) insektizide, mitizide und nematizide Mittel, die als Wirkstoff das Nitromethylen-tetrahydropyrimidin-Derivat der allgemeinen Formel (I) enthalten, und
(4) ein Verfahren zur Bekämpfung schädlicher Insekten, Milben oder Zecken und Nematoden, bei dem das Nitromethylen-tetrahydropyrimidin-Derivat der allgemeinen Formel (I) entweder allein oder in Kombination mit Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel und einem synergistischen Mittel zur Einwirkung gebracht wird.

EP 0 136 636 A2

# Nitromethylen-tetrahydropyrimidin-Derivate, Verfahren zu ihrer Herstellung sowie insektizide, mitizide, tickizide und nematizide Mittel

Die vorliegende Erfindung betrifft neue Nitromethylen-tetrahydropyrimidin-Derivate, Verfahren zu ihrer Herstellung sowie insektizide, mitizide, tickizide und nematizide Mittel, die solche Derivate als Wirkstoffe enthalten.

Insbesondere betrifft die vorliegende Erfindung neue Nitromethylen-tetrahydropyrimidin-Derivate der nachstehenden allgemeinen Formel (I)

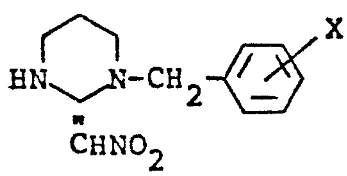

(I)

,

in der
X für Halogen steht.

Die Nitromethylen-tetrahydropyrimidin-Derivate der Formel (I) gemäß der vorliegenden Erfindung können mittels des folgenden allgemeinen Verfahrens i) hergestellt werden, auf das sich die vorliegende Erfindung ebenfalls erstreckt.

Ein Verfahren zur Herstellung der Nitromethylen-tetrahydropyrimidin-Derivate der allgemeinen Formel (I) umfaßt die Reaktion einer Verbindung der allgemeinen Formel (II)

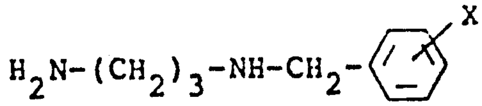

(II)

,

in der X die oben angegebene Bedeutung hat, mit 1-Nitro-2,2-bis(methylthio)ethylen der Formel

$(CH_3S)_2C{=}CHNO_2$ .

Die vorliegende Erfindung betrifft weiterhin insektizide, mitizide , tickizide und nematizide Mittel, die Nitromethylen-tetrahydropyrimidin-Derivate der Formel (I) als Wirkstoffe enthalten.

Die vor dem Anmeldezeitpunkt der vorliegenden Anmeldung bekannte DE-OS 25 14 402 stellt fest, daß 2-Nitromethylen-imidazolin-Derivate und 2-Nitromethylen-hexahydropyrimidin-Derivate der nachstehenden allgemeinen Formel

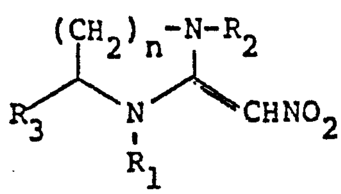

insektizide Aktivität zeigen. Die vorstehende allgemeine Formel umfaßt Fälle mit n = 2, $R_1$ = Phenyl-($C_1$-$C_2$)-alkyl-Gruppe und $R_2$ = $R_3$ = Wasserstoff, und die Beschreibung der DE-OS 25 14 402 beschreibt eine Verbindung der nachstehenden Formel:

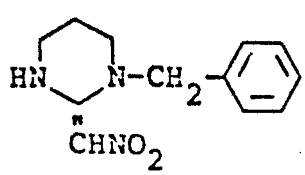

(A-1)

In der vorstehenden Formel schließt jedoch $R_1$ nicht eine Benzyl-Gruppe mit einem Substituenten ein. Nunmehr wurde gefunden, daß die neuen Nitromethylen-tetrahydropyrimidin-Verbindungen der allgemeinen Formel (I) eine hervorragende Bekämpfungswirkung gegenüber schädlichen Insekten, Milben, Zecken und Nematoden besitzen.

Die durch die obige allgemeine Formel (I) bezeichneten Nitromethylen-tetrahydropyrimidin-Derivate der vorliegenden Erfindung werden weder von der in der oben zitierten DE-OS beschriebenen allgemeinen Formel erfaßt, noch sind sie irgendwo sonst in der früher erschienenen technischen Literatur beschrieben.

Die Verbindungen der vorliegenden Erfindung sind gekennzeichnet durch die Tatsache, daß in ihrer chemischen Struktur 2-Nitromethylen-tetrahydropyrimidin als das Grundskelett vorliegt und eine halogen-substituierte Benzyl-Gruppe an dem Stickstoff-Atom in der 1-Stellung des Tetrahydropyrimidin-Rings substituiert ist. Der Phenyl-Kern ist durch Halogen vorzugsweise in der 3- oder der 4-Stellung substituiert.

Es wurde außerdem gefunden, daß die Verbindungen der vorliegenden Erfindung eine besonders hervorragende Bekämpfungswirkung gegenüber Schädlingen besitzen, die diejenige der Verbindungen der Formel (A-1), die in der oben genannten DE-OS 25 14 402 beschrieben sind und den Verbindungen der vorliegenden Erfindung am ähnlichsten sind, weit übertrifft, und daß die Verbindungen der vorliegenden Erfindung eine ausgeprägte Bekämpfungswirkung gegen Schadinsekten zeigen, die gegenüber Insektiziden vom Typ organischer Phosphate und vom Carbamat-Typ aufgrund langdauernden Einsatzes derselben Resistenz entwickelt haben, insbesondere gegen saugende Insekten wie Schaben, Laternenträger und Zikaden.

Weiterhin wurde gefunden, daß die Verbindungen der vorliegenden Erfindung besondere überlegene Bekämpfungswirkungen entfalten, wenn sie auf eine Wasseroberfläche aufgebracht werden, und in bezug auf Restwirkungen in Wasser solchen Verbindungen, die den Verbindungen der vorliegenden Erfindung sehr ähnlich sind, beispielsweise den Verbindungen der oben angegebenen Formel (A-1), weit überlegen sind.

Ein Ziel der vorliegenden Erfindung ist es, die neuen Nitromethylen-tetrahydropyrimidin-Derivate der allgemeinen Formel (I), ein Verfahren zur Herstellung derselben sowie deren Verwendung als insektizide, mitizide, tickizide und nematizide Mittel verfügbar zu machen.

Dieses sowie weitere Ziele und Vorteile der vorliegenden Erfindung werden aus der folgenden Beschreibung im einzelnen deutlich.

Die aktiven Verbindungen gemäß der vorliegenden Erfindung zeigen eine genaue Bekämpfungswirkung gegen schädliche Insekten, Milben oder Zecken und Nematoden, ohne daß sie irgendeine Phytotoxizität gegenüber Kulturpflanzen entfalten. Des weiteren können die Verbindungen der vorliegenden Erfindung zur Bekämpfung und Ausrottung einer breiten Palette von Schädlingen eingesetzt werden, darunter saugenden Insekten, beißenden Insekten und anderen Pflanzenparasiten, Schädlingen von Lagergetreide und gesundheitsgefährdenden Schädlingen.

Spezielle Beispiele für diese Schädlinge sind nachstehend aufgeführt:

Zu Beispielen für Insekten zählen

Insekten der Ordnung Coleoptera

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus.

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella
Plutella maculipennis und
Phyllocnistis citrella.

Insekten der Ordnung Hemiptera

Nephotettix cincticeps,
Nilaparvata lugens,
Pseudococcus comstocki,
Unaspis yanonensis,
Myzus persicae,
Aphis pomi,
Aphis gossypii,
Rhopalosiphum pseudobrassicas,
Stephanitis nashi,
Nazara spp.,
Cimex lectularius,
Trialeurodes vaporariorum und
Psylla spp..

Insekten der Ordnung Orthoptera

Blatella germanica,
Periplaneta americana,
Gryllotalpa africana und
Locusta migratoria migratoriodes.

Insekten der Ordnung Isoptera

Deucotermes speratus und
Coptotermes formosanus.

Insekten der Ordnung Diptera

Musca domestica,
Aedes aegypti,
Hylemia platura,
Culex pipiens,
Anopheles sinensis und
Culex tritaeniorhynchus.

<u>Milben</u>

Tetranychus telarius,
Panonychus citri,
Aculus pelekassi und
Torronomus spp..

<u>Nematoden</u>

Meloidogyne incognita,
Bursaphelenchus lignicolus Mamiya et Kiyohara,
Aphelenchoides besseyi,
Heterodera glycines und
Pratylenchus spp..

Auf dem Gebiet der Veterinärmedizin sind die neuen Verbindungen gemäß der vorliegenden Erfindung wirksam gegen verschiedene schädliche Tierparasiten (Endo- und Ektoparasiten) wie Zecken, Insekten und Würmer. Beispiele für solche Tierparasiten sind nachstehend angegeben.

<u>Zecken</u>

Oranithodoros spp.,
Ixodes spp. und
Boophilus spp..

<u>Insekten</u>

Gastrophilus spp.,
Stomoxys spp.,
Trichodectes spp.
Rhodnius spp. und
Ctenocephalidex canis

Substanzen, die eine pestizide Aktivität gegenüber all diesen Schädlingen aufweisen, werden in der vorliegenden Anmeldung gelegentlich einfach als "Insektizide" bezeichnet.

<u>Nit 1:1</u>

Die Nitromethylen-tetrahydropyrimidin-Derivate der allgemeinen Formel (I) gemäß der vorliegenden Erfindung können in einfacher Weise hergestellt werden, beispielsweise mit Hilfe des folgenden Verfahrens i).

<u>Verfahren i)</u>

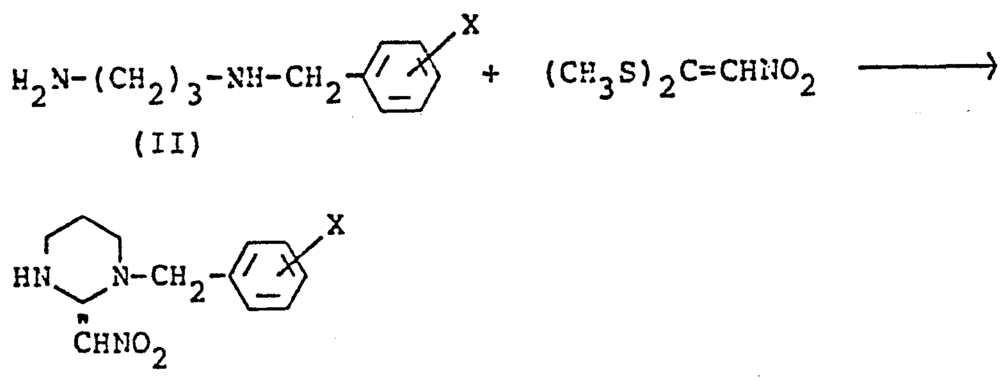

(II)

(I)

(In den Formeln hat X die im Vorstehenden angegebene Bedeutung).

In dem vorstehenden Reaktionsschema bezeichnet X ein Halogen-Atom, speziell ein Fluor-, Chlor-, Brom- oder Iod-Atom.

In dem durch das obige Reaktionsschema dargestellten Verfahren zur Herstellung der Verbindung der allgemeinen Formel (I) zählen zu speziellen Beispielen für die Ausgangs-Verbindung der allgemeinen Formel (II) N-(3-Chlorobenzyl)trimethylendiamin, N-(4-Chlorobenzyl)trimethylendiamin, N-(4-Bromobenzyl)trimethylendiamin und N-(4-Fluorobenzyl)trimethylendiamin.

Das vorgenannte Verfahren wird durch das folgende typische Beispiel im einzelnen beschrieben.

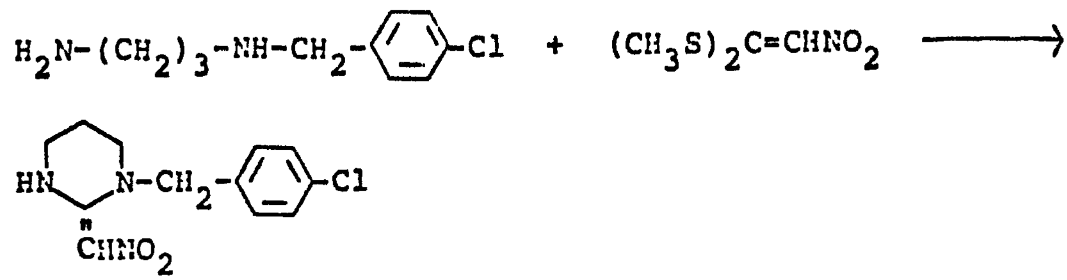

Zweckmäßigerweise kann das vorstehende Verfahren zur Herstellung der Verbindungen gemäß der vorliegenden Erfindung in einem Lösungsmittel oder Verdünnungsmittel durchgeführt werden. Zu diesem Zweck können sämtliche inerten Lösungsmittel oder Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Das obige Verfahren kann innerhalb eines breiten Temperaturbereichs durchgeführt werden. Im allgemeinen kann es bei einer Temperatur zwischen -20°C und dem Siedepunkt der Mischung, zweckmäßigerweise bei einer Temperatur zwischen etwa 0°C und etwa 100°C, durchgeführt werden. Zweckmäßigerweise wird die Reaktion unter normalem Atmosphärendruck durchgeführt, jedoch ist es ebenfalls möglich, bei erhöhtem oder vermindertem Druck zu arbeiten.

Die Verbindungen der allgemeinen Formel (I) gemäß der vorliegenden Erfindung können auch mit Hilfe eines anderen, im Folgenden schematisch dargestellten Verfahrens ii) hergestellt werden.

Verfahren ii)

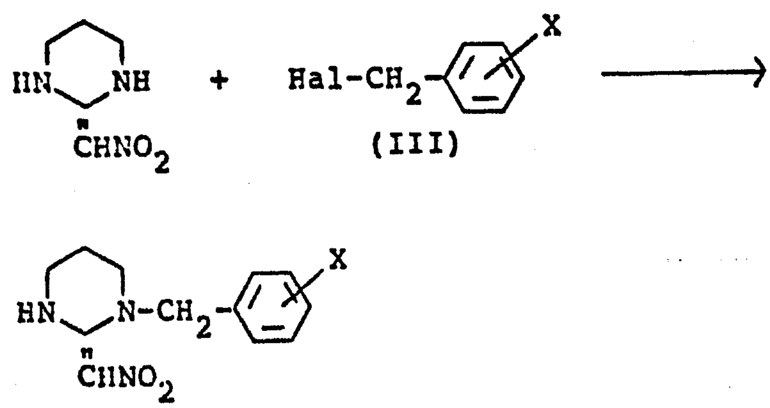

(III)

(I)

(In den Formeln hat X die im Vorstehenden angegebene Bedeutung, und Hal bezeichnet ein Halogen-Atom).

In dem vorstehenden Reaktionsschema bezeichnet X ein Halogen-Atom, speziell ein Fluor-, Chlor-, Brom- oder Iod-Atom.

In dem durch das obige Reaktionsschema dargestellten Verfahren zur Herstellung der Verbindung der allgemeinen Formel (I) zählen zu speziellen Beispielen für die Ausgangs-Verbindung der allgemeinen Formel (III) 3-Chlorobenzylchlorid, 4-Chlorobenzylchlorid, 4-Bromobenzylchlorid und 4-Fluorobenzylchlorid. Die entsprechenden Bromide sind ebenfalls zu nennen.

Das vorgenannte Verfahren ii) wird durch das folgende Bezugsbeispiel im einzelnen beschrieben.

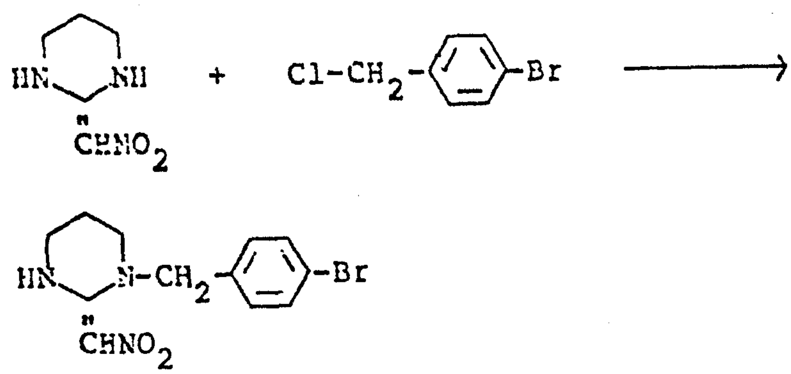

Das vorstehende Verfahren kann unter Verwendung der gleichen Lösungsmittel oder Verdünnungsmittel durchgeführt werden, wie sie im Vorstehenden beispielhaft für das Verfahren i) aufgeführt sind.

Die vorstehende Reaktion kann in Gegenwart eines säurebindenden Mittels durchgeführt werden. Beispiele für das säurebindende Mittel können die Hydroxide, Carbonate, Hydrogencarbonate und Alkoholate von Alkalimetallen sowie tertiäre Amine wie Triethylamin, Diethylanilin und Pyridin umfassen, die sämtlich im allgemeinen verwendet werden.

Nit 171

Das Verfahren ii) kann wie im Fall des Verfahrens i) innerhalb eines weiten Bereichs der Temperatur und zweckmäßigerweise unter normalem Atmosphärendruck durchgeführt werden. Es ist ebenfalls möglich, bei erhöhtem oder vermindertem Druck zu arbeiten.

Als insektizide, mitizide, tickizide und nematizide Mittel können die Verbindungen der vorliegenden Erfindung unmittelbar nach dem Verdünnen mit Wasser oder aber in Form verschiedenartiger Formulierungen zur Anwendung gebracht werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden diese verschiedenen Formulierungen entweder unmittelbar oder nach dem Verdünnen mit Wasser auf die gewünschte Konzentration eingesetzt.

Beispiele für die hierin erwähnten landwirtschaftlich unbedenklichen Hilfsstoffe sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /¯z.B. n-Hexan, Petrolether, Erdöl-Fraktionen (z.B. Paraffinwachse, Kerosin, Leichtöle, Mittelöle und Schweröle), Benzol, Toluol und Xylole_7, halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas (LNG) und niedere Ether), die Verbrennung steuernde Mittel für Räuchermittel (z.B. Nitrite, Zink-Pulver und Dicyandiamid), sauerstoff-abgebende Mittel (z.B. Chlorate), wirkungsverlängernde Mittel, Dispersions-Stabilisatoren (z.B. Casein, Tragant, Carboxymethylcellulose (CMC) und Polyvinylalkohol (PVA)) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate, benetzbare Pulver, lösliche Pulver, Suspensionen, Stäubemittel, Granulate, pulvrige Präparate, Räuchermittel, Tabletten, Aerosole, Pasten und Kapseln.

Die insektiziden, mitiziden oder nematiziden Mittel gemäß der vorliegenden Erfindung können etwa 0,1 bis etwa 95 Gew.-%, vorzugsweise etwa von 0,5 bis 90 Gew.-% des vorerwähnten Wirkstoffes enthalten.

Für den praktischen Gebrauch beträgt die geeignete Menge der aktiven Verbindung in den vorgenannten verschiedenartigen Formulierungen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art der Formulierung, dem Verfahren, Zweck, der Zeit und dem Ort seiner Anwendung sowie dem Zustand des Auftretens der zu bekämpfenden schädlichen Insekten, Milben oder Zecken und Nematoden variiert werden.

Erforderlichenfalls können die Verbindungen gemäß der vorliegenden Erfindung weiterhin auch in Kombination mit anderen Agrochemikalien verwendet werden, beispielsweise mit anderen Insektiziden, Fungiziden, anderen Mitiziden, anderen Nematiziden, Anti-Virus-Mitteln, Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, Organochlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen_7 und/oder Düngemitteln.

Den im Vorstehenden bezeichneten Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Spritzen, Bedampfen, Gießen etc.), Oberflächen-Anwendung (z.B. Beschichten, Aufbringen in Form von Bändern, Pulverbeschichten, Bedecken etc.), Eintauchen und Ködern. Sie können auch mittels des sogenannten Ultra-Low-Volume-Sprühverfahrens aufgebracht werden. Nach diesem Verfahren kann der Wirkstoff sogar in einer Konzentration von 100 % eingearbeitet sein.

Die Aufwandmenge pro Flächeneinheit beträgt beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein insektizides, mitizides, tickizides oder nematizides Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung von Insekten, Milben oder Zecken und Nematoden verfügbar, das darin besteht, daß eine Verbindung der allgemeinen Formel (I) allein oder in Form einer Mischung mit einem Verdünnungsmittel (einem Lösungsmittel und/ oder einem Streckmittel und/oder einem Träger) und/oder einem oberflächenaktiven Mittel und, falls weiterhin erforderlich, einem Stabilisator, einem Haftmittel, einem synergistischen Mittel etc. auf schädliche Insekten, Milben oder Zecken und Nematoden und/oder deren Lebensraum oder den Ort ihres Auftretens aufgebracht wird.

Die vorliegende Erfindung wird durch die folgenden Beispiele im einzelnen erläutert, ist jedoch nicht auf diese speziellen Beispiele allein beschränkt.

Beispiel 1

N-(4-Chlorobenzyl)trimethylendiamin (8,45 g) und 1-Nitro-2,2-bis(methylthio)ethylen (6,60 g) wurden in 100 ml Ethanol 16 h unter Rückfluß erhitzt. Die Reaktionsmischung wurde auf Raumtemperatur abgekühlt, und die ausgefallenen Kristalle wurden durch Filtration gesammelt und mit einer kleinen Menge Ethanol gewaschen, wonach 1-(4-Chlorobenzyl)-2-(nitromethylen)-tetrahydropyrimidin (9,8 g) der nachstehenden Formel erhalten wurde. Schmelzpunkt: 172-174°C.

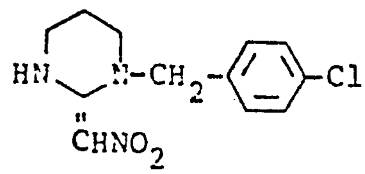

(Verbindung Nr. 1)

Die nachstehende Tabelle 1 führt die Verbindungen der vorliegenden Erfindung auf, die aus den entsprechenden Ausgangsstoffen in gleicher Weise wie in Beispiel 1 synthetisiert wurden.

Tabelle 1

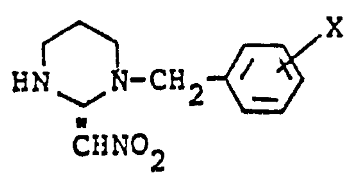

| Verbindung Nr. | X | Physikal. Konstante (Schmp.,°C) |
|---|---|---|
| 2 | 3-Cl | 190-192 |
| 3 | 4-Br | 198-199 |
| 4 | 4-F | 171-173 |

Nit 171

Beispiel 2 (Benetzbares Pulver)

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf schädliche Insekten, Milben oder Nematoden und/oder ihren Lebensraum oder den Ort ihres Auftretens aufgesprüht.

Beispiel 3 (Emulgierbares Konzentrat)

30 Teile der Verbindung Nr. 3 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf schädliche Insekten, Milben oder Nematoden und/oder ihren Lebensraum oder den Ort ihres Auftretens aufgesprüht.

Beispiel 4 (Stäubemittel)

2 Teile der Verbindung Nr. 4 der Erfindung und 98 Teile Tonpulver werden pulverisiert und gemischt, wodurch ein Stäubemittel hergestellt wird. Dieses wird über schädlichen Insekten, Milben oder Nematoden und/oder ihrem Lebensraum oder dem Ort ihres Auftretens ausgestreut.

Beispiel 5 (Granulat)

Wasser (25 Teile) wird zu einer Mischung aus 10 Teilen der Verbindung Nr. 2 der Erfindung, 30 Teilen Bentonit

(Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird, wodurch ein Granulat gebildet wird. Das Granulat wird über schädlichen Insekten, Milben oder Nematoden und/oder ihrem Lebensraum oder dem Ort ihres Auftretens ausgestreut.

Beispiel 6 (Granulat)

Ein Drehmischer wird mit 95 Teilen Tonmineral-Teilchen mit einer Teilchengrößen-Verteilung zwischen 0,2 und 2 mm beschickt, und unter Drehen des Mischers werden 5 Gew.-Teile der Verbindung Nr. 3 der Erfindung auf die Tonmineral-Teilchen zur gleichmäßigen Benetzung derselben aufgesprüht. Die feuchte Mischung wird bei 40°C bis 50°C getrocknet, wodurch ein Granulat gebildet wird. Das Granulat wird über schädlichen Insekten, Milben oder Nematoden und/oder ihrem Lebensraum oder dem Ort ihres Auftretens ausgestreut.

Beispiel 7 (Öl-Präparat)

Die Verbindung Nr. 1 der Erfindung (0,5 Teile) und 99,5 Teile Kerosin werden unter Rühren miteinander vermischt, wodurch ein Öl-Präparat gebildet wird. Dieses wird auf schädliche Insekten, Milben oder Nematoden und/oder ihren Lebensraum oder den Ort ihres Auftretens aufgesprüht.

Beispiel 8 (Biologischer Test)

Test mit gegen Organophosphor-Mittel resistenten Nephotettix cincticeps:

Herstellung eines Test-Präparats:

| | | |
|---|---|---|
| Lösungsmittel: | Xylol | 3 Gew.-Teile |
| Emulgator: | Polyoxyethylen-alkylphenylether | 1 Gew.-Teil |

Zur Herstellung eines geeigneten Test-Präparats wurde 1 Gew.-Teil der aktiven Verbindung mit der oben bezeichneten Menge Lösungsmittel, das die oben angegebene Menge Emulgator enthielt, vermischt, und die Mischung wurde mit Wasser auf eine vorher festgesetzte Konzentration verdünnt.

Test-Verfahren:
Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Wirkstoff-Konzentration aufweisenden Lösungen, die wie oben angegeben hergestellt wurden, gesprüht. Die aufgesprühte Chemikalie wurde trocknen gelassen, und über die Reispflanzen wurde ein Drahtkorb von 7 cm Durchmesser und 14 cm Höhe gestülpt, unter dem 30 ausgewachsene weibliche Exemplare von Nephotettix cincticeps, die gegen Organophosphor-Insektizide resistent waren, ausgesetzt wurden. Die Töpfe wurden jeweils in einem Raum mit konstanter Temperatur aufbewahrt, und 24 Stunden später wurde die Zahl der toten Insekten bestimmt und das Vernichtungsverhältnis berechnet.

Die Ergebnisse sind in Tabelle 2 aufgeführt.

Tabelle 2

| Verbindung Nr. | Wirkstoff-Konzentration ppm | Vernichtungs-verhältnis % |
|---|---|---|
| 1 | 8 | 100 |
| 2 | 8 | 100 |
| 3 | 8 | 100 |
| 4 | 8 | 100 |
| Vergleichs-Verbindung A-1 | 40 | 60 |

Anmerkungen zu Tabelle 2:

1) Die Verbindungs-Nummern sind die gleichen, wie sie oben angegeben sind.

2) Die Vergleichs-Verbindung A-1 ist die Verbindung der folgenden Formel, die im Vorstehenden genannt wurde.

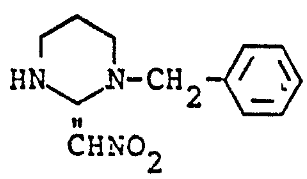

0136636

Beispiel 9 (Biologischer Test)

Test mit gegen Organophosphor-Mittel resistenten Myzodes persicae (grünen Pfirsichblattläusen):

Test-Verfahren:
Gezüchtete grüne Pfirsichblattläuse wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 8 hergestellte wäßrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28°C stehen gelassen. 24 Stunden nach dem Sprühen wurde das Vernichtungsverhältnis berechnet. Für jede Verbindung wurde der Test als Doppelbestimmung durchgeführt.

Die Ergebnisse sind in Tabelle 3 aufgeführt.

Tabelle 3

| Verbindung Nr. | Wirkstoff-Konzentration ppm | Vernichtungs-verhältnis % |
|---|---|---|
| 1 | 200 | 100 |
| 3 | 200 | 100 |
| 4 | 200 | 100 |
| Vergleichs-Verbindung A-1 | 1000 | 80 |
| | 200 | 30 |
| Estox (Handelsprodukt) | 1000 | 100 |
| | 200 | 20 |

Anmerkungen zu Tabelle 3:

1) Die Verbindungs-Nummern und die Vergleichs-Verbindung A-1 sind die gleichen, wie sie oben angegeben sind.

2) Estox: S-2-Ethylsulfinyl-1-methylethyldimethylphosphorothiolat (45-proz. emulgierbares Konzentrat).

## P a t e n t a n s p r ü c h e

1. Nitromethylen-tetrahydropyrimidin-Derivat der allgemeinen Formel

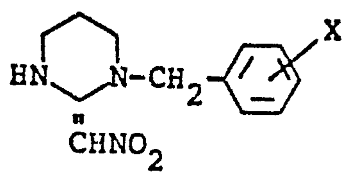

,

in welcher X für Halogen steht.

2. Verbindung nach Anspruch 1, dadurch gekennzeichnet, daß X für ein in 4-Stellung des Phenyl-Kerns gebundenes Halogen-Atom steht.

3. 1-(4-Chlorobenzyl)-2-(nitromethylen)tetrahydropyrimidin nach Anspruch 1 oder Anspruch 2 der Formel:

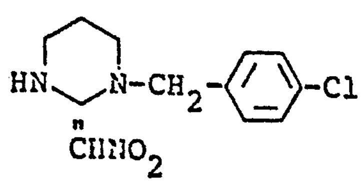

4. 1-(4-Bromobenzyl)-2-(nitromethylen)tetrahydropyrimidin nach Anspruch 1 oder Anspruch 2 bezeichnet durch die der Formel:

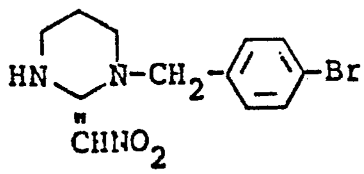

5. Verfahren zur Herstellung eines Nitromethylen-tetrahydropyrimidin-Derivats der allgemeinen Formel

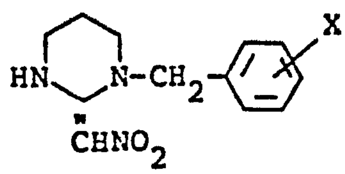

,

in der X für Halogen steht,
dadurch gekennzeichnet, daß

a) eine Verbindung der allgemeinen Formel

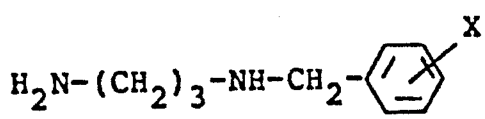

,

in der X die oben angegebene Bedeutung hat, mit 1-Nitro-2,2-bis(methylthio)ethylen der Formel

$(CH_3S)_2C=CHNO_2$

umgesetzt wird oder

b) eine Verbindung der allgemeinen Formel

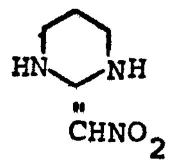

mit einer Verbindung der allgemeinen Formel

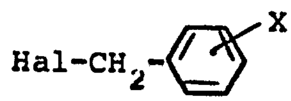

,

in der X die oben angegebene Bedeutung hat, umgesetzt wird.

6. Insektizides, mitizides, tickizides und nematizides Mittel, enthaltend als Wirkstoff ein Nitromethylen-tetrahydropyrimidin-Derivat der allgemeinen Formel

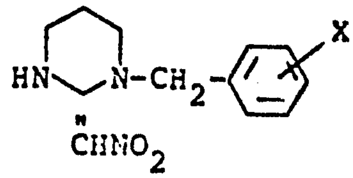

,

in der X für Halogen steht.

7. Verfahren zur Bekämpfung schädlicher Insekten, Milben oder Zecken und Nematoden, dadurch gekennzeichnet, daß das Nitromethylen-tetrahydropyrimidin-Derivat der allgemeinen Formel (I) entweder allein oder in Kombination mit Verdünnungsmittel und/oder einem oberflächenaktiven Mittel und, falls weiterhin erforderlich, einem Stabilisator, einem Haftmittel und einem synergistischen Mittel zur Einwirkung gebracht wird.